Europäisches Patentamt

European Patent Office

Office européen des brevets

(11) Publication number: **0 233 759**
A1

## (12) EUROPEAN PATENT APPLICATION

(21) Application number: **87301234.8**

(22) Date of filing: **12.02.87**

(51) Int. Cl.³: **C 07 C 67/38**
**C 07 C 69/54, C 07 C 69/24**

(30) Priority: **13.02.86 GB 8603556**

(43) Date of publication of application:
**26.08.87 Bulletin 87/35**

(84) Designated Contracting States:
**BE DE FR GB IT NL**

(71) Applicant: **The British Petroleum Company p.l.c.**
**Britannic House Moor Lane**
**London EC2Y 9BU(GB)**

(72) Inventor: **Preece, Michael The British Petroleum Co. p.l.c.**
**Chertsey Road Sunbury-on-Thames**
**Middlesex, TW16 7LN(GB)**

(72) Inventor: **Smith, David J. H. The British Petroleum Co. p.l.c**
**Chertsey Road Sunbury-on-Thames**
**Middlesex, TW16 7LN(GB)**

(74) Representative: **Richardson, Derek et al,**
**BP INTERNATIONAL LIMITED Patents & Agreements**
**Division Chertsey Road**
**Sunbury-on-Thames Middlesex TW16 7LN(GB)**

(54) Process for the production of a mixture of carboxylic acid esters.

(57) A mixture of an acrylate and a propionate ester is produced by reacting under substantially anhydrous conditions a feedstock mixture comprising ethylene, acetylene, an alcohol and carbon monoxide at elevated temperature, preferably less than 110°C, in the presence of a catalyst consisting essentially of a carbonyl of a metal of Group VIII of the Periodic Table of the Elements, particularly rhodium. The ethylene, acetylene and carbon monoxide is preferably obtained by partial oxidation and simultaneous cracking of a saturated hydrocarbon feedstock.

EP 0 233 759 A1

# PROCESS FOR THE PRODUCTION OF A MIXTURE OF CARBOXYLIC ACID ESTERS

The present invention relates generally to a process for the production of a mixture of carboxylic acid esters and in particular to the production of a mixture of propionate and acrylate esters.

Acrylate esters are used in the production of emulsion and solution polymers for a wide variety of applications including coatings, finishes, binders, paints, floor polishes and adhesives. Propionate esters are also useful chemical products.

Processes are known for the production of mixtures of carboxylic acids from for example US-A-4,111,982 and EP-A-96974.

US Patent No. 4,111,982 discloses a process wherein propionic acid and acetic acid are produced simultaneously and continuously by contacting feed components comprising methanol, ethylene and water in a molar ratio from about 1:5:5 to about 100:1:1 together with carbon monoxide in a single reactor with a liquid reaction medium and a catalyst system consisting essentially of a rhodium compound and a halogen component which is bromine, iodine, a bromide or an iodide, said contacting being effected at temperatures from about 50°C to about 300°C and at partial pressures of carbon monoxide from 1kg/cm$^2$ to 1056kg/cm$^2$, controlling the rate of introduction of said methanol feed at or below:

$$5 \times 10^{10}e^{-7830/T}[R][I] \text{ gram-moles/litre-hr}$$

where

T   = reactor temperature, °Kelvin

[R] = molarity of rhodium in the reactor, and

[I] = molarity of iodine in the reactor,

1

continuously withdrawing a portion of the liquid reaction mass from said reactor and separating therefrom acetic and propionic acids.

European application publication No. 0096974 describes a process for the production of a mixture of carboxylic acid anhydrides by reacting (i) an olefin, and (ii) an alcohol, with (iii) carbon monoxide at elevated temperature in the presence of a catalyst containing rhodium or iridium and a promoter comprising a halogen selected from bromine and iodine in free or combined form. The mixed anhydrides are convertible by reaction with water to the mixed carboxylic acids.

It would be desirable to utilise widely available chemical feedstocks, such as ethylene, acetylene and carbon monoxide, which are obtainable by a variety of routes, and in particular it would be desirable to produce acrylate and propionate esters from gaseous paraffinic hydrocarbons, such as those which comprise natural gas, of which there are large reserves throughout the world.

In this connection Japanese Laid-Open Patent Application No. 57-11928 discloses a method of manufacturing ethylene and a gas having hydrogen and carbon monoxide as its main components (synthesis gas feedstock) by partially burning methane using an oxygen-containing gas to produce a gas having acetylene, hydrogen and carbon monoxide as its main components, passing the gas so-produced over a hydrogenation catalyst and semi-hydrogenating acetylene in the gas to give ethylene, separating ethylene from the mixed gas and refining the remainder, so giving pure ethylene and synthesis gas feedstock for chemicals production.

We have now surprisingly found that ethylene, acetylene and carbon monoxide can be utilised for the production of a mixture of acrylate and propionate esters, and moreover that the product from the partial oxidation of a gaseous paraffinic hydrocarbon can be utilised for the production of the aforesaid product without the need to hydrogenate the acetylene to ethylene.

Accordingly, the present invention provides a process for the production of a mixture of an acrylate and a propionate ester by reacting under substantially anhydrous conditions a feedstock mixture comprising ethylene, acetylene, an alcohol and carbon monoxide at elevated temperature in the presence of a catalyst consisting essentially of a carbonyl of a metal of Group VIII of the Periodic Table of the Elements or the precursors thereof.

The carbon monoxide, ethylene and acetylene may suitably be substantially pure or may contain impurities such as hydrogen, carbon dioxide, nitrogen, water or methane.

Suitable Group VIII metals include iron, cobalt, nickel, rhodium, iridium, platinum, ruthenium, palladium and osmium. Preferred Group VIII metals include rhodium and iridium, more preferred is rhodium. The metal carbonyl is preferably a binary carbonyl. Polynuclear carbonyls are also preferred. It is essential for the performance of the invention that the metal carbonyl is used in the absence of halogen promoters or quaternary nitrogen, phosphorus or arsenic copromoters, which are often found to be beneficial in carbonylation processes. The metal carbonyl may be added as such or in the form of its precursors and the metal carbonyl generated 'in situ'. The metals may be used in the zero valent state or in any higher valent form. Thus the metal may be added as the elemental metal in finely divided form, as a simple salt such as the halide or as an organometallic compound such as a carboxylate salt.

Examples of rhodium and iridium compounds which may be added include $Rh_6(CO)_{16}$ and $Ir_4(CO)_{12}$.

The alcohol may suitably be a lower aliphatic alkanol, for example methanol, ethanol or isopropanol. The alcohol dictates the nature of the product acrylate and propionate esters.

We have found that in order to produce acrylates and propionates, it is necessary to use substantially anhydrous conditions. It may therefore be necessary to dry some or all of the reactants before use in the process of the invention.

The catalyst may be supported, i.e. it may be dispersed on a conventional support material, such as for example alumina, silica/alumina, zeolites, clays, titania or zirconia. The catalyst may be applied to the support in conventional manner, e.g. by impregnation from solution. The catalyst concentration on the support may suitably be in the range from 0.01 to 10% by weight. Alternatively, the metal catalyst components may be attached to an ion exchange resin or to a functionalised inorganic oxide such as those described in the complete specification of GB 1503315 or European patent publication No. 18102 (BP Case No. 4752).

As regards the amounts of the various reactants which may be employed, the ratios of reactants may be varied over a wide range depending on the ratio of the individual products desired.

The elevated temperature is preferably maintained below about 110°C, more preferably in the range from 50 to 100°C.

The partial pressure of carbon monoxide and olefin (when the olefin is gaseous) may suitably be in the range from 1.0 to 1000 bar, preferably from 10 to 100 bar. Higher pressures may be used if so desired under appropriate conditions.

The process may be operated in the liquid phase or in the vapour phase, preferably in the liquid phase. In the liquid phase a solvent may be employed if desired. Suitable solvents include both aliphatic or aromatic hydrocarbons. A preferred solvent is a monocarboxylic acid having from 2 to 20 carbon atoms or a mixture thereof.

The process may be operated batchwise or continuously, preferably continuously.

The individual esters may be recovered from the product mixture by means well-known in the art.

A mixture comprising acetylene, ethylene and carbon monoxide for use as feedstock may suitably be obtained by partial oxidation of a saturated hydrocarbon under conditions whereby the saturated hydrocarbon is simultaneously cracked to form unsaturated hydrocarbons.

In a preferred embodiment, a mixture comprising acetylene, ethylene and carbon monoxide for use as feedstock mixture is produced by a process in which (a) a saturated hydrocarbon and an oxygen-containing gas having a ratio of hydrocarbon to oxygen of greater than the stoichiometric ratio for complete combustion are introduced into a bed of inert particulate material, (b) the upward flow rate of the hydrocarbon/oxygen-containing gas stream being sufficiently large to fluidise or to produce a spouting action of the bed material, whereby at least a part of the particulate material is thrown up above the bed surface and subsequently falls back into the bed, (c) the hydrocarbon and oxygen-containing gas being ignited and reacted together and (d) the products of the reaction being withdrawn. This process is described in our copending European application publication No. 0163385 (BP Case No. 5791) to which the reader is referred for further details.

In a particularly preferred embodiment of the present invention a mixture comprising acetylene, ethylene and carbon monoxide for use as feedstock is produced by a process in which (a) a saturated hydrocarbon and an oxygen-containing gas having a ratio of hydrocarbon to oxygen of greater than the stoichiometric ratio for complete combustion are introduced together with hydrogen into a slumped bed of particulate material (b) the upward flow rate or the gases being sufficiently large to fluidise or to cause a spouting action of the bed material above the slumped level of the bed (c) the hydrocarbon, oxygen and hydrogen reacting together and (d) the products of the reaction being withdrawn. This process is further described in our copending European application publication No. 0178853 (BP Case No. 5959) to which the reader is referred for further detail.

The product of the aforesaid processes may, depending upon the manner in which the processes are operated, contain hydrogen, aromatic hydrocarbons, water and unreacted methane in addition to acetylene, ethylene and carbon monoxide. The product may, without any intervening treatment, other than drying and possibly adjustment of its temperature and pressure, be combined with alcohol, and optionally also additional carbon monoxide, and contacted with the catalyst as aforesaid. If desired, the ratio of acetylene:ethylene may be adjusted before contact with the catalyst.

The invention will now be further illustrated by reference to the following Examples.

In the Examples a feedstock mixture consisting of acetylene, ethylene, carbon monoxide and hydrogen in the ratio 6:3:30:61 by volume simulating a product as shown in Tables 1 and 2 of EP-A-178853 (BP Case No. 5959) was used.

Analysis of the final reaction mixture was performed by gas chromatography/mass spectrometry.

Example 1

To a 100 ml (nominal volume) stainless steel autoclave was added toluene (25 mls), methanol (1 ml) and $Rh_6(CO)_{16}$ (0.05 g). The system was pressurised to 10 bar with a gas mixture comprising acetylene, ethylene, carbon monoxide, hydrogen (6:3:30:61 by vol), and then vented; this was repeated twice and the autoclave finally pressurised to 9.1 bar with the same gas mixture. The autoclave was heated to 60°C and maintained at this temperature for 24 hours. Analysis of the final reaction mixture indicated that it contained methyl acrylate (0.6% w/w), methyl propionate (0.2% w/w), 1,1-dimethoxypropane (1.0% w/w) and propanal (0.08% w/w).

Example 2

The procedure of Example 1 was repeated except that the temperature was 80°C, the pressure was 10.6 bar and the reaction time was 2 hours.

Analysis of the final reaction solution indicated that it contained 1,1-dimethoxypropane (0.8% w/w), methyl acrylate (0.02% w/w) and methyl propionate (0.02% w/w).

Example 3

The procedure of Example 1 was repeated except that the temperature was 100°C.

Analysis of the final reaction solution indicated that it contained dimethoxypropane (1.4% w/w), methyl propionate (0.1% w/w) and methyl acrylate (0.05% w/w).

Example 4

The procedure of Example 1 was repeated except that the temperature was 80°C.

Analysis of the final reaction solution indicated that it contained methyl propionate (0.1% w/w), methyl acrylate (0.07% w/w)

and 1,1-dimethoxypropane (1.5% w/w).

Comparison Test 1

The procedure of Example 1 was repeated except that triphenylphosphine (0.80g) was added to the initial charge.

Analysis of the final reaction mixture indicated that it contained propanal (0.9% w/w), acetone (0.01% w/w) with traces of methylethylketone and methyl propionate.

Comparison Test 2

The procedure of Example 1 was repeated except that TDA (1 ml) was added to the initial charge and the pressure was 7.1 bar.

Analysis of the final reaction solution indicated that it contained propanal (0.1% w.w).

Comparison Test 3

The procedure of Example 1 was repeated except that water (0.45 g) was added to the initial reaction mixture instead of methanol.

Analysis of the final reaction solution indicated that there was no detectable amount of acrylic and propionic acids.

Comparison Test 4

Comparison Test 3 was repeated except that the reaction temperature was 100°C and the pressure was 9.8 bar.

Analysis of the final reaction solution indicated that it contained 1,4-dioxan (0.4% w/w) and propanal (0.7% w/w).

Comparison Tests 1-4 are not according to the present invention and are included only for the purpose of comparison.

The results of the Comparison Tests demonstrate that in order to produce acrylate and propionate esters the conditions must be substantially anhydrous and conventional promoters must be absent.

Claims:

1 A process for the production of a mixture of an acrylate and a propionate ester by reacting under substantially anhydrous conditions a feedstock mixture comprising ethylene, acetylene, an alcohol and carbon monoxide at elevated temperature in the presence of a catalyst consisting essentially of a carbonyl of a metal of Group VIII of the Periodic Table of the Elements or the precursors thereof.

2 A process according to claim 1 wherein the metal carbonyl is a binary carbonyl.

3 A process according to claim 1 wherein the metal carbonyl is a polynuclear carbonyl.

4 A process according to any one of the preceding claims wherein the Group VIII metal is rhodium.

5 A process according to any one of the preceding claims wherein the alcohol is a lower aliphatic alkanol.

6 A process according to any one of the preceding claims wherein the temperature is in the range from 50 to 110°C.

7 A process according to any one of the preceding claims wherein a mixture comprising acetylene, ethylene and carbon monoxide for use as feedstock mixture is obtained by partial oxidation of a saturated hydrocarbon under conditions whereby the saturated hydrocarbon is simultaneously cracked to form unsaturated hydrocarbons.

8 A process according to any one of the preceding claims wherein a mixture comprising acetylene, ethylene and carbon monoxide for use as feedstock mixture is produced by a process in which (a) a saturated hydrocarbon and an oxygen-containing gas having a ratio of hydrocarbon to oxygen of greater than the stoichiometric ratio for complete combustion are introduced into a bed of inert particulate material, (b) the upward flow rate of the hydrocarbon/oxygen-containing gas stream being sufficiently large to fluidise or to produce a spouting action of the bed material, whereby at least a part of the particulate material is thrown up above the bed surface and subsequently falls back into the bed, (c) the hydrocarbon and oxygen-containing gas being ignited and reacted together and (d) the products of the reaction being withdrawn.

9 A process according to any one of claims 1 to 7 wherein a mixture comprising acetylene, ethylene and carbon monoxide for use as feedstock mixture is produced by a process in which (a) a saturated hydrocarbon and an oxygen-containing gas having a ratio of hydrocarbon to oxygen of greater than the stoichiometric ratio for complete combustion are introduced together with hydrogen into a slumped bed of particulate material (b) the upward flow rate of the gases being sufficiently large to fluidise or to cause a spouting action of the bed material above the slumped level of the bed (c) the hydrocarbon, oxygen and hydrogen reacting together and (d) the products of the reaction being withdrawn.

10 A process according to either claim 8 or claim 9 wherein the mixture comprising acetylene, ethylene and carbon monoxide is without any intervening treatment, other than drying and possibly adjustment of its temperature and pressure, combined with alcohol, and optionally also additional carbon monoxide, and used as the feedstock mixture.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | DE-A-1 011 415 (BASF)<br><br>* Column 1, lines 21-27; column 1, line 42 - column 2, line 34; column 2, line 42 - column 3, line 24 *<br><br>--- | 1,5,7, 10 | C 07 C 67/38<br>C 07 C 69/54<br>C 07 C 69/24 |
| Y | FR-A-2 184 938 (MONSANTO)<br>* Page 3, line 33 - page 4, line 6; page 4, line 23 - page 5, line 17; page 7, lines 2-19; page 17; claims 1,7 *<br><br>--- | 1-6 | |
| Y | DE-A-2 948 888 (ROHM)<br>* Page 1, claims 1,2; page 4 line 23 - page 5, line 7 *<br><br>--- | 1,4,5 | |
| Y | US-A-3 060 228 (P. PINO)<br><br>* Column 1, lines 39-60; columns 2,3, example 1 *<br><br>--- | 1,2,5, 6 | **TECHNICAL FIELDS SEARCHED (Int. Cl. 4)**<br><br>C 07 C 69/00<br>C 07 C 67/00 |
| Y | FR-A-1 459 733 (TOA GOSEI KAGAKU KABUSHIKI KAISHA)<br>* Page 5, example 1; page 7, claims A-1,A-2,A-7 *<br><br>----- | 1,5,6 | |

| | | |
|---|---|---|
| The present search report has been drawn up for all claims | | |
| Place of search<br>THE HAGUE | Date of completion of the search<br>15-05-1987 | Examiner<br>KINZINGER J.M. |

EPO Form 1503 03 82

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document